# EUROPEAN PATENT APPLICATION

(11) **EP 1 739 459 A1**
(43) Date of publication of application: **03.01.2007**
(21) Application number: 06011642.3
(22) Date of filing: 06.06.2006
(51) Int. Cl.: G01T 1/29

(54) **Computerized tomography system**

(30) Priority: 08.06.2005 JP 2005167826
(71) Applicant: HITACHI, LTD., Chiyoda-ku Tokyo 100-8280 (JP)
(72) Inventor: Kitazawa, So, Hitachi Ltd., chome Chiyoda-ku Tokyo 100-8220 (JP); Kamimura, Hiroshi Hitachi Ltd., chome Chiyoda-ku Tokyo 100-8220 (JP); Nukaga, Jun, Hitachi Ltd., chome Chiyoda-ku Tokyo 100-8220 (JP)
(74) Representative: Strehl Schübel-Hopf & Partner

(57) **Abstract**

The present invention provides a CT system capable of obtaining tomographic images having high image quality regardless of a change in the ambient temperature for a long period of time even if a cooling device is simple.

Computerized tomography system, comprising: a detecting device to detect radiation rays that penetrate through an object to be detected; and a collimator to restrict radiation rays entering the detecting device, wherein a separation gap for thermally separating the collimator and the detecting device is defined between an end surface (6f) of the collimator (6) at the detecting device side and an end surface (22f) of the a detector holder at the collimator side in the detecting device , and the separation gap is hermetically sealed.

## Description

### BACKGROUND OF THE INVENTION

### [FIELD OF THE INVENTION]

The present invention relates to a computerized tomography system, and more particularly to a computerized tomography system suitable for a case in which high-energy radiation rays are employed for an industrial purpose.

### [Prior Art]

In general, a computerized tomography system (CT system) is designed in such a manner that radiation rays that have been irradiated from a source of radiation (radiation source) such as an X-ray tube or a linear accelerator and penetrate an object to be detected are detected by plural detectors that are arranged linearly or two-dimensionally, and a tomographic image of the object to be detected is obtained by an image reconstruction from output data of the respective detectors. In the CT system of this type, there occurs such a phenomenon that radiation rays from various directions are entered to the detectors as scattered rays that have been scattered by the object to be detected in addition to the radiation rays that linearly penetrate the object to be detected from the radiation source.

The image quality of the obtained tomographic image is deteriorated by the phenomenon that the radiation rays are entered to the detectors from the unspecified directions. For that reason, it is general that the CT system is equipped with a collimator for restricting the radiation rays incident upon the detectors. The collimator includes a precollimator and a postcollimator.

The precollimator is disposed in the vicinity of the radiation source so as to restrict the irradiated range of the radiation rays that occur in the radial pattern from the radiation source to a desired range, to thereby reduce the radiation rays that are leaked to the external of the CT system.

On the other hand, the postcollimator is disposed immediately in front of the detectors so as to decrease a solid angle of the radiation source from the detectors, thereby preventing the scattered rays caused by the object to be detected from being entered to the detectors and blocking the radiation rays that are emitted from portions other than a true focal point of the radiation source to restrict the radiation rays that are entered to the detectors. In other words, the postcollimator mainly functions to prevent the radiation rays from being entered to the detectors from the unspecified directions so as to improve the image quality.

The postcollimator has a structure in which plural penetration slits each having a minute size which is, for example, several tens cm in the length and 1 mm or less in the width. The plural slits are so disposed as to correspond to the arrangement of the detectors for passage of the radiation rays toward the respective detectors. As a result, the postcollimator is required to be positioned with a precision of 0.1 mm order with respect to the detectors, more specifically a detector holder that holds plural detectors in a given arrangement state. From the above viewpoint, it is general that the postcollimator and the detector holder are integrated together, or the postcollimator and the detector holder are clamped against each other by some means and connected to each other.

As described above, in the CT system, the collimators are involved in the image quality of the tomographic image, but the image quality of the tomographic image also suffers from a problem on the leakage current of the detectors. For example, in the X-ray CT system, it is general that a detector into which a scintillator and a photo diode are combined together, or a detector that is formed of a single photo diode is employed as the detector.

In the detector into which the scintillator and the photo diode are combined together, the X-rays are converted into a visible light or a light having wavelengths close to those of the visible light by the scintillator, and the converted light is converted into electric charges by a photodiode and then extracted as a signal.

On the other hand, in the detector formed of the single photo diode, the X-rays that are entered to the photodiode are converted directly into electric charges and extracted as a signal. A silicon photo diode is generally employed as the photo diode, but other photo diodes than the silicon photo diode may be used. Even in the detector that is formed of the single photo diode other than the silicon photo diode, the entererd X-rays are converted directly into electric charges and then extracted as signals as in the silicon photo diode.

In the detector of this type, it is general that non-bias or reverse bias of several tens volts is applied, and in any cases, the current is not zero ideally even in a state where the X-rays are not entered at all, and a leakage current of several pA to several nA flows. The leakage current has a temporal fluctuation on the basis of the Poisson statistics of electrons, which is one of factors that deteriorate a signal to noise ratio (S/N) of the image quality.

Also, the leakage current changes depending on the bias that is applied to the photo diode, and a temperature state of the photo diode, and the change of the leakage current is also one factor that deteriorates the quality of image. For that reason, it is desirable to reduce the leakage current as small as possible.

A representative example of the deterioration of the image quality which is caused by the leakage current is a ring artifact (virtual image of a concentric pattern) in the X-ray CT image that has been picked up by the third generation system. The X-ray CT image pickup method includes the respective systems of the first generation, the second generation, and the third generation from the viewpoint of historic development background, and the second generation system and the third generation system are mainly employed in an industrial X-ray CT system.

The third generation system is a system that acquires transmission data of one tomography while the object to be detected is rotating by 360 degrees. The third generation system has the advantage that the image pickup time is the shortest in the respective systems but has disadvantage that the ring artifact is liable to appear in the image attributably to the individual difference of the X-ray sensitivities of the respective detectors.

It is usual to cope with the ring artifact by a manner in which coefficients for correcting the individual difference of the respective detectors are experimentally obtained, and the individual difference is adjusted by the coefficients so that the ring artifact does not appear. However, in the above coping process, the temperatures of the detectors change with time. As a result , in the case where the leakage current changes, and the correcting effect is low, there arises such a problem that the ring artifact is liable to appear. It is difficult to eliminate this problem.

In order to solve the ring artifact problem that is caused by the temperature change of the detectors, there is frequently used a method of keeping a constant temperature of the detectors through a temperature adjusting mechanism. Because there has been theoretically known that an absolute value of the leakage current and a change amount thereof become smaller as the temperature is lower, the ring artifact problem can be effectively prevented by keeping the constant temperatures of the detectors, which is lower than the room temperature. In this case, it is general that the whole detector holder is cooled, and the detectors are held to a constant low temperature.

The above-described CT systems are disclosed in, for example, Patent Document 1 to Patent Document 5.
[Patent Document 1] Japanese Application Patent Laid-open Publication No. 2003-130819.
[Patent Document 2] Japanese Application Patent Laid-open Publication No. Hei 11-304929.
[Patent Document 3] Japanese Application Patent Laid-open Publication No. 2002-34968.
[Patent Document 4] Japanese Application Patent Laid-open Publication No. Hei 11-271456.
[Patent Document 5] Japanese Application Patent Laid-open Publication No. 2001-153959A

### SUMMARY OF THE INVENTION

### [PROBLEMS TO BE SOLVED BY THE INVENTION]

As described above, the CT system suffers from the ring artifact problem that is attributable to the leakage current depending on the temperature state in the detectors, and copes with the problem by the method of keeping the constant temperature of the detectors through the temperature adjustment mechanism.

In the conventional CT system, as described above, the postcollimator is integrated with the detector holder, or the postcollimator and the detector holder are connected to each other in a state where they are clamped against each other. For that reason, a heat that flows into the detectors due to the thermal conduction from the postcollimator that is large in the heat capacity is large, and a cooling device that is large in the cooling performance is required in order to cool the detectors and keep the constant low temperature of the cooled detectors.

For example, in the case of the X-ray CT system, it is necessary to provide a cooling mechanism that circulates, within the detector holder, a coolant water that has been cooled by a cooling device that is located at the external of the X-ray CT system. For that reason, the cooling device becomes large in the size, and a large area at which the cooling device is located is required. Also, the cooling device becomes expensive, and the cyclic maintenance of the cooling device is required. Thus, the conventional CT system suffers from various problems.

The above problems are particularly remarkable in the industrial X-ray CT system. In other words, in the industrial X-ray CT system, the energy of the radiation rays is large, which causes the postcollimator to also increase in the size. As a result, the heat capacity of the postcollimator becomes also large, and the amount of heat that flows into the detectors from the postcollimator becomes large, to thereby further upsize the cooling device.

Also, the radiation source that generates the radiation rays of a high energy is driven by a direct current, which causes the leakage current to be large with the result that the conventional X-ray CT system is liable to be influenced by the leakage current. In addition, in the industrial X-ray CT system, there are many cases in which very high quality of image is required, and the cooling device is not prevented from being upsized in order to meet such a requirement.

Under the above circumstances, a structure has been strongly desired in which in the CT system, particularly, the industrial CT system, the detectors are held to a constant low temperature even by a simple cooling device.

The present invention has been made to meet the above requirement, and therefore an object of the present invention is to provide CT system that is capable of obtaining tomographic images that are high in the image quality regardless of a change in the ambient temperature for a long period of time even if a cooling device is sample.

### [MEANS FOR SOLVING THE PROBLEMS]

In order to achieve the above object, according to the present invention, there is provided a computerized tomography system, comprising a detecting device that detects radiation rays that penetrates an object to be detected; and a collimator that restricts radiation rays that are entered to the detecting device, wherein a separation gap that thermally separates the collimator and the detecting device is defined between an end surface of the collimator at the detecting device side and an end surface of the detecting device at the collimator side, and the separation gap is hermetically sealed.

Also, according to the present invention, in the computerized tomography system, the end surface of the detecting device which faces the separation gap is covered with a thermal insulating coating.

Also, according to the present invention, in order to achieve the above object, there is provided a computerized tomography system, comprising a detecting device that detects radiation rays that penetrates an object to be detected; and a collimator that restricts radiation rays that are entered to the detecting device, wherein the detecting device is coated with a thermal insulating coating to define a cooling gap in the periphery of the detecting device.

Further, in order to achieve the above object, according to the present invention, there is provided a computerized tomography system, comprising: a detecting device that detects radiation rays that penetrates an object to be detected; and a collimator that restricts radiation rays that are ntered to the detecting device, wherein a separation gap that thermally separates the collimator and the detecting device is defined between an end surface of the collimator at the detecting device side and an end surface of the detecting device at the collimator side, and the separation gap is hermetically sealed, and wherein the detecting device is coated with a thermal insulating coating to define a cooling gap in the periphery of the detecting device.

### [EFFECTS OF THE INVENTION]

In the present invention, the separation gap that thermally separates the collimator and the detecting device is defined between the collimator and the detecting device, and the separation gap is hermetically sealed. With this structure, according to the present invention, it is possible to effectively prevent thermal effects of the collimator that is large in the thermal capacity from reaching the detecting device. As a result, it is easy to maintain the temperature of the detecting device, it is possible to keep the constant temperature of the detecting device with a high precision even if a simple cooling device is used, and it is possible to obtain a tomographic image with a high image quality for a long period of time regardless of a change in the ambient temperature.

Also, in the present invention, the detecting device is covered with the thermal insulating coating to define the cooling gap in the periphery of the detecting device. The cooling gap functions to more efficiently and uniformly cool the detecting device through the cooling device. For that reason, according to the present invention, it is possible to more efficiently cool the detecting device, it is possible to keep the constant temperature of the detecting device with a high precision even if the simple cooling device is used, and it is possible to obtain a tomographic image with a high image quality for a long period of time regardless of a change in the ambient temperature.

### BRIEF DESCRIPTION OF THE DRAWINGS

Fig. 1 is a plan view schematically showing a structure of an X-ray CT system according to an embodiment.
Fig. 2 is a front view showing the structure of the X-ray CT system shown in Fig. 1.
Fig. 3 is a vertical cross-sectional view showing a main portion of the X-ray CT system shown in Fig. 1.
Fig. 4 is a cross-sectional view taken along a dash-dot line in Fig. 3.
Fig. 5 is a diagram showing a structure of a positioning and gap setting spacer.

### DETAILED DESCRIPTION OF THE INVENTION

### [DESCRIPTION OF THE PREFERRED EMBODIMENTS]

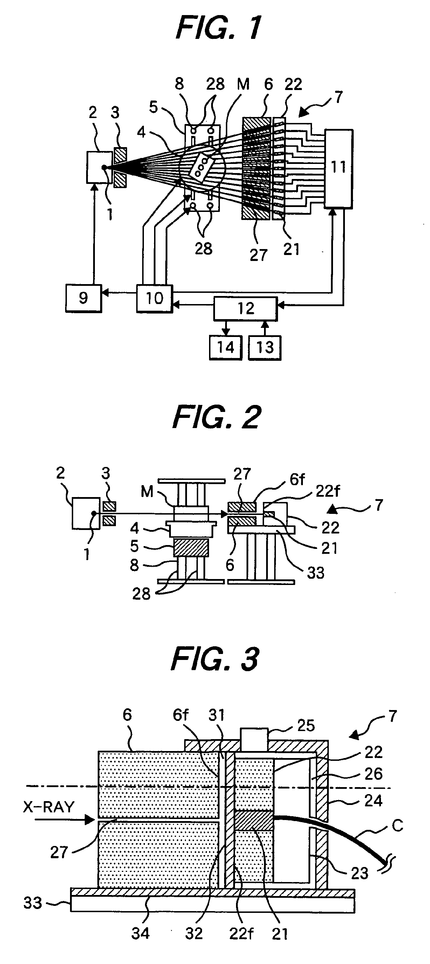

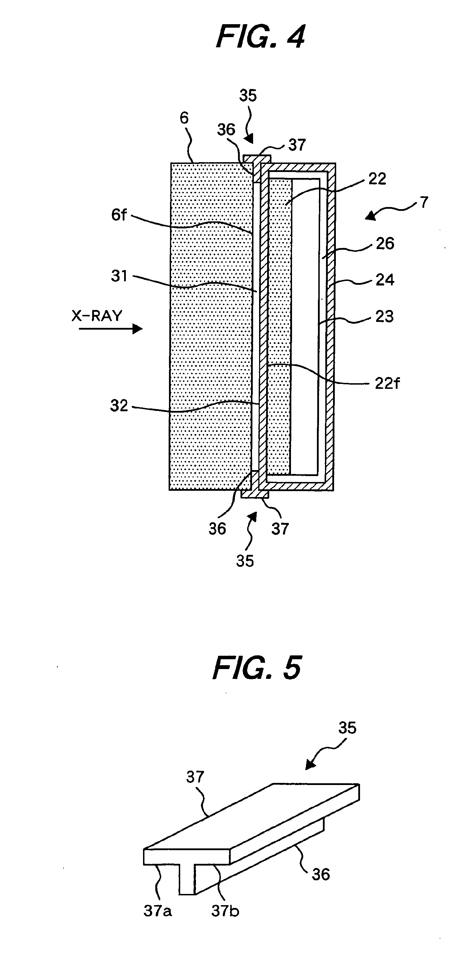

The following describes an embodiment of the present invention. Figs. 1 to 4 schematically show the structure of an X-ray CT system which is a CT system according to one embodiment. Fig. 1 shows the structure viewed from a plan direction, and Fig. 2 shows the structure viewed from a front direction. Figs. 3 and 4 are enlarged diagrams showing the main portion of the X-ray CT system. Fig. 3 shows across-sectionof themainportion along a vertical direction, and Fig. 4 shows a cross-section of Fig. 3 taken along a dash-dot line. As shown in those figures, the X-ray CT system according to this embodiment includes an X-ray source device 2 having an X-ray source 1, a precollimator 3, a rotary table 4, a translation motion device 5, a postcollimator 6, a detecting device 7, a rotary table up/down device 8, an X-ray source control device 9, a driving mechanism control device 10, a detector circuit 11, a calculator 12, an input device 13, and a display device 14.

The X-ray source device 2 allows the X-ray source 1 to generate X-rays, and radiates the X-rays. The X-ray source control device 9 controls the radiation and suspension of radiation of the X-rays in the X-ray source device 2. The X-ray source 1 of the X-ray source device 2 is formed of, for example, an X-ray tube, or an electron linear accelerator.

The precollimator 3 is disposed immediately downstream of the X-ray source device 2, and functions to restrict an irradiated range of X-rays that are generated from the X-ray source 1 in a radial fashion to a desired range, in this example, to a fan beam shape (fan shape). For that reason, the precollimator 3 is made of a material that is high in the X-ray shielding function such as lead or tungsten, and has a window for shaping the X-rays in the fan beam.

The detecting device 7 allows a detector holder 22 to hold plural detectors 21 that are arranged in a line, and a protective case 23 (Fig. 3) to support the detector holder 22. The detecting device 7 is covered with a thermal insulating coating 24, and cooled by a cooling device 25 to keep a constant low temperature of the detectors 21. As shown in Fig. 1, the respective detectors 21 are arranged at given angle pitches with respect to the X-ray source 1. Each of the detectors 21 is formed of a detector comprising a scintillator and a photo diode, or a semiconductor detector made of Si, CdTe, CdZn, Te, HgI2, or GaAs. The detector holder 22 is frequently made of a metal such as copper, brass, or stainless steel for facilitation of machining, but may be made of a nonmetal such as plastic or ceramic. The protective case 23 is so structured as to support the detector holder 22 in a state where only an end surface (a surface from which the radiation rays are entered to the detectors 21) 22f of the detector holder 22 at the postcollimator 6 side (Figs. 2 and 3) are exposed. The thermal insulating coating 24 is made of a material that is remarkably small in the thermal conductivity as compared with a metal material of the detector holder 22 or the protective case 23, for example, a hard resin material. The thermal insulating coating 24 is structured to cover the detecting holder 7 so as to define a cooling gap 26 with an appropriate width with respect to the detecting device 7, more specifically, to the detector holder 22 or the protective case 23.

The structure in which the cooling gap 26 is defined in the periphery of the detecting device 7 as described above is one of the features of the present invention, and the cooling gap 26 functions to more efficiently and uniformly cool the plural detectors 21 that are held by the detector holder 22 by the cooling device 25. In other words, air in the cooling gap 26 which is cooled by the cooling device 25 and kept to a constant temperature uniformly cools the plural detectors 21 through the protective case 23 or the detector holder 22, thereby making it possible to more efficiently and uniformly conduct cooling. For that reason, even if a simple cooling device such as an electron cooling device or cooling device using the Peltier effect is employed as the cooling device 25, it is possible to keep the constant temperature of the detector 21 with a high precision, and it is possible to obtain the tomographic image of a high image quality with no ring artifact for a long period of time regardless of a change in the ambient temperature. This becomes more effectively by combining the separation structure of the detecting device 7 and the postcollimator 6 which will be described later.

In this example, as shown in Fig. 3, a cable C for signals is connected to the detectors 21. For that reason, cable lead holes are appropriately defined in the protective case 23 and the thermal insulating coating 24, and the cable C is taken out to the external through the cable lead holes.

The postcollimator 6 is disposed in the vicinity of the detecting device 7, and has a function of preventing the radiation rays scattered by an object M to be detected on the rotary table 4 from being entered to the detectors 21, and shielding the X-rays that are radiated from portions other than a focal point of the X-ray source 1. As a result, the postcollimator 6 is made of a material that is high in the X-ray shielding performance such as lead or tungsten, and radiation passage slits 27 of the same number as that of the detectors 21 are defined in an arrangement corresponding to the arrangement of the detectors 21 in the detecting device 7. Each of the slits 27 is fine, that is, several tens cm in the length and 1 mm or less in the width, and is directed toward the X-ray source 1 so as to determine the X-ray directions that are entered to the detectors 21.

The rotary table 4 has a function of conducting the motion of the object to be detected M which is required during image pickup of the object to be detected M mounted on the rotary table 4, and also conducts the translation motion through the translation motion device 5. Also, the rotary table 4 moves up/down through the up/down motion of the translation motion device 5 by the rotary table up/down device 8. The rotary table up/down device 8 has four rotary screws 28 that extend vertically and are engaged with the table of the translation motion device 5. Those rotary screws 28 are rotated by a motor to move up/down the translation motion device 5.

The driving operation of the respective driving mechanisms of the rotary table 4, the translation motion device 5, and the rotary table up/down device 8 is controlled according to a control signal that is outputted from a driving mechanism control device 10. The driving mechanism control device 10 generates pulses every predetermined rotary angles when the rotary table 4 rotates. The pulses functions as trigger signals for X-ray generation, and also as gate signals for taking in detection signals from the respective detectors 21 by the detector circuit 11. The pulses that function as the trigger signals for X-ray generation are transmitted to the X-ray source control device 9 from the driving mechanism control device 10. The pulses that function as the gate signals for taking in the detection signals from the respective detectors 21 are transmitted to the detector circuit 11 from the drivingmechanism control device 10.

The detector circuit 11 amplifies the detection analog signals that are outputted from the respective detectors 21, and thereafter converts the analog signals to digital signals. Then, the detector circuit 11 outputs the digital signals to the calculator 12. The calculator 12 reconstructs the tomographic image through a given process on the basis of the inputted detection signals. The tomographic image thus obtained is displayed on the display device 14, and can be appropriately printed by a printer (not shown).

Hereinafter, a description will be given of the separation structure of the postcollimator 6 and the detecting device 7 which is another feature of the present invention. As shown in Figs. 3 and 4, a separation gap 31 of an appropriate width is defined between an end surface 6f the postcollimator 6 at the detecting device 7 side (a surface from which the radiation rays that have passed through the slit 27 are outputted) and an end surface 22f of the detector holder 22. In other words, the postcollimator 6 and the detecting device 7 face each other through the separation gap 31, and are not in direct contact with each other, that is, are thermally isolated from each other by the separation gap 31. Also, the periphery of the separation gap 31 is sealed to make the separation gap 31 in a hermetically sealed state.

More specifically, a portion of the thermal insulating coating 24 which is covered the detecting device 7 is elongated so that the periphery of the separation gap 31 is sealed by the elongated portion of the thermal insulating coating 24 to make the separation gap 31 in a hermetically sealed state.

In addition, in this embodiment, the end surface 22f of the detector holder 22 which faces the separation gap 31 is covered with a thermal insulating coating 32 that is made of a hard resin material which is small in the thermal conductivity as with the thermal insulating coating 24.

It is preferable that the width of the separation gap 31 is set to about several mm. In the example shown in the figures, the width of the separation gap 31 is set to 4 mm, and the thickness of the thermal insulating coating 32 is set to 3 mm.

As described above, there is provided the separation structure in which the hermetically sealed separation gap 31 is defined between the postcollimator 6 and the detecting device 7, thereby making it possible to remarkably reduce the heat that flows into the detector holder 22 from the postcollimator 6. More specifically, in the case of the separation structure, the heat that flows into the detector holder 22 from the postcollimator 6 comprises only a component caused by the thermal conduction through air in the separation gap 31, and a component caused by the thermal conduction through a support table 33. The heat that flows into the detector holder 22 through air in the separation gap 31 can be remarkably reduced since the separation gap 31 is hermetically sealed so as to suppress a convective conduction caused by air convection in the separation gap 31 as much as possible, and the end surface 22f of the detector holder 22 which faces the separation gap 31 is covered with the thermal insulating coating 32. Also, the heat that flows into the detector holder 22 through the support table 33 can be remarkably reduced because a thermal insulating plate 34 like the thermal insulating coating 24 and the thermal insulating coating 32 is interposed between the support table 33 and the postcollimator 6 and between the support table 33 and the detecting device 7.

As a result, it is possible to effectively prevent the thermal effect caused by the postcollimator 6 which is large in the thermal capacity from reaching the detectors 21. As a result, it becomes easy to keep the temperature of the detectors 21. Even if a simple cooling device is employed as the cooling device 25, it is possible to keep the constant temperature of the detector 21 with a high precision, and it is possible to obtain the tomographic image of a high image quality with no ring artifact for a long period of time regardless of a change in the ambient temperature.

As described above, in order to provide the structure in which the postcollimator 6 and the detecting device 7 are separated from each other, it is necessary to consider the positioning of the postcollimator 6 and the detecting device 7. To meet this necessity, it is effective to position the postcollimator 6 and the detecting device 7 while the separation gap 31 is set by using a positioning and gap setting spacer 35. The positioning and gap setting spacer 35 is made of the same material as that of the thermal insulating coating 24 and the thermal insulating coating 32. Also, as enlarged in Fig. 5, the positioning and gap setting spacer 35 is structured by combining a gap setting spacer portion 36 with a positioning abutting portion 37 in such a manner that those portions 36 and 37 are orthogonal to each other. The positioning and gap setting spacer 35 is as small as possible in the size. The positioning of the postcollimator 6 and the detecting device 7 by means of the positioning and gap setting spacer 35 is conducted as follows. The gap setting spacer portion 36 is held between the end surface 6f of the postcollimator 6 and the end surface 22f of the detector holder 22 that is covered with the thermal insulating coating 32. A side surface of the postcollimator 6 is abutted against an abutment surface 37a of the positioning abutting portion 37, and a side surface of the detecting device 7 (a side surface of the thermal insulating coating 24 that covers the detecting device 7 in the drawing) is abutted against an abutment surface 37b of the positioning abutting portion 37 to fix the positional relationship between the postcollimator 6 and the detecting device 7. As a result, the high-precision positioning is conducted even if the postcollimator 6 and the detecting device 7 are separated from each other. The positioning and gap setting spacer 35 may be removed from the system after the positioning has been completed.

According to the present invention, in the CT system, even if a simple cooling device is employed, it is possible to obtain the tomographic image of a high image quality for a long period of time regardless of a change in the ambient temperature, and the CT system can be widely employed in a field such as the X-ray CT system.

## Claims

1. Computerized tomography system comprising a detecting device to detect radiation rays being penetrated an object to be detected, and a collimator to restrict radiation rays to be entered to the detecting device,
wherein a separation gap for separating thermally the collimator and the detecting device is defined between an end surface of the collimator at the detecting device side and an end surface of the detecting device at the collimator side, and a periphery of the separation gap is hermetically sealed.

2. Computerized tomography system according to claim 1, wherein the end surface of the detecting device being faced the separation gap is covered with a thermal insulating coating.

3. Computerized tomography system comprising a detecting device to detect radiation rays being penetrated an object to be detected and a collimator to restrict radiation rays to be entered to the detecting device,
wherein the detecting device is coated with a thermal insulating coating to define a cooling gap in the periphery of the detecting device.

4. Computerized tomography system comprising a detecting device to detect radiation rays being penetrated an object to be detected and a collimator to restrict radiation rays to be entered to the detecting device,
wherein a separation gap for separating thermally the collimator and the detecting device is defined between an end surface of the collimator at the detecting device side and an end surface of the detecting device at the collimator side, and a periphery of the separation gap is hermetically sealed, and the detecting device is coated with a thermal insulating coating to define a cooling gap in the periphery of the detecting device.
